(19) Europäisches Patentamt
European Patent Office
Office européen des brevets

(11) EP 1 550 873 A1

(12) EUROPEAN PATENT APPLICATION

(43) Date of publication:
**06.07.2005 Bulletin 2005/27**

(51) Int Cl.[7]: **G01N 33/573**, C12N 9/16

(21) Application number: **03380310.7**

(22) Date of filing: **30.12.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicants:
- **Progenika Biopharma, S.A.**
  **48160 Derio (Vizcaya) (ES)**
- **UNIVERSIDAD DEL PAIS VASCO**
  **48940 Leioa (Vizcaya) (ES)**

(72) Inventors:
- **Ariz Lopez de Castro, Usue**
  **48160 Derio Vizcaya (ES)**
- **Osaba Ortiz de Mendibil, Lourdes**
  **48160 Derio Vizcaya (ES)**
- **Junquera Sanchez-Vallejo, Corina**
  **48160 Derio Vizcaya (ES)**
- **Ochoa Garay, Jorge**
  **48160 Derio Vizcaya (ES)**
- **Escuredo Garcia de Galdeano, Pedro**
  **48160 Derio Vizcaya (ES)**
- **Santa Cruz, Simon**
  **48160 Derio Vizcaya (ES)**
- **Simon Buela, Laureano**
  **48160 Derio Vizcaya (ES)**
- **Martinez Martinez, Antonio**
  **48160 Derio Vizcaya (ES)**
- **MatuteAlmau, Carlos**
  **48940 Leioa Vizcaya (ES)**
- **Domercq Garcia, Maria**
  **48940 Leioa Vizcaya (ES)**
- **Alberdi Alfonso, Elena**
  **48940 Leioa Vizcaya (ES)**
- **Victoria Sanchez Gomez, Maria**
  **48940 Leioa Vizcaya (ES)**
- **Vallejo Illarramendi, Ainara(2)**
  **48940 Leioa Vizcaya (ES)**
- **Ibarretxe Bilbao, Gaskon (2)**
  **48940 Leioa Vizcaya (ES)**
- **Etxebarria Galnares, Estibaliz (2)**
  **48940 Leioa Vizcaya (ES)**

(74) Representative: **Portela Gasch, Sergio Raul**
**ABG Patentes, S.L.,**
**Orense, 16-8.o A**
**28020 Madrid (ES)**

(54) **Methods for the in vitro diagnosis and prognosis of demyelinating diseases and for the development of drugs against demyelinating diseases**

(57) The present invention refers to an in vitro method for detecting the presence of demyelinating diseases in an individual, for determining the stage or severity of said diseases in the individual, or for monitoring the effect of the therapy administered to an individual suffering said diseases; to the search, identification, development and evaluation of efficacy of compounds for therapy of said diseases for the purpose of developing new drugs; as well as to agents inhibiting DUSP6 protein expression and/or activity, and/or the effects of this expression. The methods and agents of the invention are preferably applied to multiple sclerosis.

EP 1 550 873 A1

SEQUENCE LISTING

<110> MEDPLANT GENETICS, S.L.; UNIVERSIDAD DEL PAÍS VASCO

<120> Methods for the in vitro diagnosis and in vitro prognosis of demyelinating diseases, and for the development of drugs against demyelinating diseases

<160> 25

<170> PatentIn version 2.0

<210> 1
<211> 22
<212> DNA
<213> artificial sequence

<220> synthetic DNA
<223> direct primer designed to amplify, in combination with SEQ ID NO: 2, cDNA of the rat dusp6 gene

<400> 1
gggagagatt tgctccattc at 22

<210> 2
<211> 23
<212> DNA
<213> artificial sequence

<220> synthetic DNA
<223> reverse primer designed to amplify, in combination with SEQ ID NO: 1, cDNA of the rat dusp6 gene

<400> 2
aaaagcaaac ctattgcctg gat 23

<210> 3
<211> 20
<212> DNA
<213> artificial sequence

<220> synthetic DNA
<223> dusp6 gene antisense oligonucleotide ODN2

<400> 3
tcaacgtggc catcccgggc 20

<210> 4
<211> 21
<212> DNA
<213> artificial sequence

<220> synthetic DNA
<223> dusp6 gene antisense oligonucleotide ODN3

<400> 4
ccaagtggac tccctgcaat c 21

<210> 5

<211> 25
<212> DNA
<213> artificial sequence

<220> synthetic DNA
<223> probe sequence of the U42627_at probe set of Affymetrix, the position of said probe in the mRNA sequence of the dusp6 gene being 1557

<400> 5
ttcagtttct cttgggcagc atcga 25

<210> 6
<211> 25
<212> DNA
<213> artificial sequence

<220> synthetic DNA
<223> probe sequence of the U42627_at probe set of Affymetrix, the position of said probe in the mRNA sequence of the dusp6 gene being 1563

<400> 6
ttctcttggg cagcatcgac caggc 25

<210> 7
<211> 25
<212> DNA
<213> artificial sequence

<220> synthetic DNA
<223> probe sequence of the U42627_at probe set of Affymetrix, the position of said probe in the mRNA sequence of the dusp6 gene being 1623

<400> 7
gtcaccagct gtctgtatta gacaa 25

<210> 8
<211> 25
<212> DNA
<213> artificial sequence

<220> synthetic DNA
<223> probe sequence of the U42627_at probe set of Affymetrix, the position of said probe in the mRNA sequence of the dusp6 gene being 1713

<400> 8
ggacagggta tgctgtctag atcca 25

<210> 9
<211> 25
<212> DNA
<213> artificial sequence

<220> synthetic DNA
<223> probe sequence of the U42627_at probe set of Affymetrix, the position of said probe in the mRNA sequence of the dusp6 gene being 1725

<400> 9
ctgtctagat ccaggcaata ggttt 25

<210> 10
<211> 25
<212> DNA
<213> artificial sequence

<220> synthetic DNA
<223> probe sequence of the U42627_at probe set of Affymetrix, the position of said probe in the mRNA sequence of the dusp6 gene being 1773

<400> 10
agcagggact ggacctccat ccaga 25

<210> 11
<211> 25
<212> DNA
<213> artificial sequence

<220> synthetic DNA
<223> probe sequence of the U42627_at probe set of Affymetrix, the position of said probe in the mRNA sequence of the dusp6 gene being 1827

<400> 11
ggagcatgtg ttccttaggg ccaca 25

<210> 12
<211> 25
<212> DNA
<213> artificial sequence

<220> synthetic DNA
<223> probe sequence of the U42627_at probe set of Affymetrix, the position of said probe in the mRNA sequence of the dusp6 gene being 1845

<400> 12
ggccacatat ggctgtttcc tgttg 25

<210> 13
<211> 25
<212> DNA
<213> artificial sequence

<220> synthetic DNA
<223> probe sequence of the U42627_at probe set of Affymetrix, the position of said probe in the mRNA sequence of the dusp6 gene being 1857

<400> 13
ctgtttcctg ttgcatctgg aacca 25

<210> 14
<211> 25
<212> DNA
<213> artificial sequence

<220> synthetic DNA
<223> probe sequence of the U42627_at probe set of Affymetrix, the position of said probe in the mRNA sequence of the dusp6 gene being 1863

<400> 14
cctgttgcat ctggaaccaa ctata 25

<210> 15
<211> 25
<212> DNA
<213> artificial sequence

<220> synthetic DNA
<223> probe sequence of the U42627_at probe set of Affymetrix, the position of said probe in the mRNA sequence of the dusp6 gene being 1881

<400> 15
aactatattg tcttcagtga agact 25

<210> 16
<211> 25
<212> DNA
<213> artificial sequence

<220> synthetic DNA
<223> probe sequence of the U42627_at probe set of Affymetrix, the position of said probe in the mRNA sequence of the dusp6 gene being 1887

<400> 16
attgtcttca gtgaagactg attca 25

<210> 17
<211> 25
<212> DNA
<213> artificial sequence

<220> synthetic DNA
<223> probe sequence of the U42627_at probe set of Affymetrix, the position of said probe in the mRNA sequence of the dusp6 gene being 1935

<400> 17
gagattttag ctctgtattt gtggt 25

<210> 18
<211> 25
<212> DNA
<213> artificial sequence

<220> synthetic DNA
<223> probe sequence of the U42627_at probe set of Affymetrix, the position of said probe in the mRNA sequence of the dusp6 gene being 1941

<400> 18
ttagctctgt atttgtggta tcggt 25

<210> 19
<211> 25
<212> DNA
<213> artificial sequence

<220> synthetic DNA
<223> probe sequence of the U42627_at probe set of Affymetrix, the position of said probe in the mRNA sequence of the dusp6 gene being 2007

<400> 19
aatatttgat cttcacttga gagtg 25

<210> 20
<211> 25
<212> DNA
<213> artificial sequence

<220> synthetic DNA
<223> probe sequence of the U42627_at probe set of Affymetrix, the position of said probe in the mRNA sequence of the dusp6 gene being 2013

<400> 20
tgatcttcac ttgagagtgt ttgtt 25

<210> 21
<211> 20
<212> DNA
<213> artificial sequence

<220> synthetic DNA
<223> direct primer designed to amplify, in combination with SEQ ID NO: 22, cDNA of the rat glyceraldehyde 3-phosphate dehydrogenase gene

<400> 21
aaggctgggg ctcacctgaa 20

<210> 22
<211> 21
<212> DNA
<213> artificial sequence

<220> synthetic DNA
<223> reverse primer designed to amplify, in combination with SEQ ID NO: 21, cDNA of the rat glyceraldehyde 3-phosphate dehydrogenase gene

<400> 22
ggcatggact gtggtcatga g 21

<210> 23
<211> 20
<212> DNA
<213> artificial sequence

<220> synthetic DNA
<223> a dusp6 gene antisense oligonucleotide

<400> 23
cgttgagcca cgccaccgtc 20

<210> 24
<211> 23
<212> DNA
<213> artificial sequence

<220> synthetic DNA
<223> primer used in quantitative real-time RT-PCR for evaluating the expression of dusp6 gene in post-mortem human tissue samples from optic nerve

<400> 24

cctgaggcca tttctttcat aga 23

<210> 25
<211> 23
<212> DNA
<213> artificial sequence

<220> synthetic DNA
<223> primer used in quantitative real-time RT-PCR for evaluating the expression of dusp6 gene in post-mortem human tissue samples from optic nerve

<400> 25
gtcacagtga ctgagcggct aat 23

**SEQUENCE LISTING**

<110> MEDPLANT GENETICS, S.L.; UNIVERSIDAD DEL PAÍS VASCO

<120> Methods for the in vitro diagnosis and in vitro prognosis of demyelinating diseases, and for the development of drugs against demyelinating diseases

<160> 25

<170> PatentIn version 2.0

<210> 1
<211> 22
<212> DNA
<213> artificial sequence

<220> synthetic DNA
<223> direct primer designed to amplify, in combination with SEQ ID NO: 2, cDNA of the rat dusp6 gene

<400> 1
gggagagatt tgctccattc at 22

<210> 2
<211> 23
<212> DNA
<213> artificial sequence

<220> synthetic DNA
<223> reverse primer designed to amplify, in combination with SEQ ID NO: 1, cDNA of the rat dusp6 gene

<400> 2
aaaagcaaac ctattgcctg gat 23

<210> 3
<211> 20
<212> DNA
<213> artificial sequence

<220> synthetic DNA
<223> dusp6 gene antisense oligonucleotide ODN2

<400> 3
tcaacgtggc catcccgggc 20

<210> 4
<211> 21
<212> DNA
<213> artificial sequence

<220> synthetic DNA
<223> dusp6 gene antisense oligonucleotide ODN3

<400> 4
ccaagtggac tccctgcaat c 21

<210> 5

<211> 25
<212> DNA
<213> artificial sequence

<220> synthetic DNA
<223> probe sequence of the U42627_at probe set of Affymetrix, the position of said probe in the mRNA sequence of the dusp6 gene being 1557

<400> 5
ttcagtttct cttgggcagc atcga 25

<210> 6
<211> 25
<212> DNA
<213> artificial sequence

<220> synthetic DNA
<223> probe sequence of the U42627_at probe set of Affymetrix, the position of said probe in the mRNA sequence of the dusp6 gene being 1563

<400> 6
ttctcttggg cagcatcgac caggc 25

<210> 7
<211> 25
<212> DNA
<213> artificial sequence

<220> synthetic DNA
<223> probe sequence of the U42627_at probe set of Affymetrix, the position of said probe in the mRNA sequence of the dusp6 gene being 1623

<400> 7
gtcaccagct gtctgtatta gacaa 25

<210> 8
<211> 25
<212> DNA
<213> artificial sequence

<220> synthetic DNA
<223> probe sequence of the U42627_at probe set of Affymetrix, the position of said probe in the mRNA sequence of the dusp6 gene being 1713

<400> 8
ggacagggta tgctgtctag atcca 25

<210> 9
<211> 25
<212> DNA
<213> artificial sequence

<220> synthetic DNA
<223> probe sequence of the U42627_at probe set of Affymetrix, the position of said probe in the mRNA sequence of the dusp6 gene being 1725

<400> 9
ctgtctagat ccaggcaata ggttt 25

<210> 10
<211> 25
<212> DNA
<213> artificial sequence

<220> synthetic DNA
<223> probe sequence of the U42627_at probe set of Affymetrix, the position of said probe in the mRNA sequence of the dusp6 gene being 1773

<400> 10
agcagggact ggacctccat ccaga 25

<210> 11
<211> 25
<212> DNA
<213> artificial sequence

<220> synthetic DNA
<223> probe sequence of the U42627_at probe set of Affymetrix, the position of said probe in the mRNA sequence of the dusp6 gene being 1827

<400> 11
ggagcatgtg ttccttaggg ccaca 25

<210> 12
<211> 25
<212> DNA
<213> artificial sequence

<220> synthetic DNA
<223> probe sequence of the U42627_at probe set of Affymetrix, the position of said probe in the mRNA sequence of the dusp6 gene being 1845

<400> 12
ggccacatat ggctgtttcc tgttg 25

<210> 13
<211> 25
<212> DNA
<213> artificial sequence

<220> synthetic DNA
<223> probe sequence of the U42627_at probe set of Affymetrix, the position of said probe in the mRNA sequence of the dusp6 gene being 1857

<400> 13
ctgtttcctg ttgcatctgg aacca 25

<210> 14
<211> 25
<212> DNA
<213> artificial sequence

<220> synthetic DNA
<223> probe sequence of the U42627_at probe set of Affymetrix, the position of said probe in the mRNA sequence of the dusp6 gene being 1863

<400> 14
cctgttgcat ctggaaccaa ctata 25

<210> 15
<211> 25
<212> DNA
<213> artificial sequence

<220> synthetic DNA
<223> probe sequence of the U42627_at probe set of Affymetrix, the position of said probe in the mRNA sequence of the dusp6 gene being 1881

<400> 15
aactatattg tcttcagtga agact 25

<210> 16
<211> 25
<212> DNA
<213> artificial sequence

<220> synthetic DNA
<223> probe sequence of the U42627_at probe set of Affymetrix, the position of said probe in the mRNA sequence of the dusp6 gene being 1887

<400> 16
attgtcttca gtgaagactg attca 25

<210> 17
<211> 25
<212> DNA
<213> artificial sequence

<220> synthetic DNA
<223> probe sequence of the U42627_at probe set of Affymetrix, the position of said probe in the mRNA sequence of the dusp6 gene being 1935

<400> 17
gagattttag ctctgtattt gtggt 25

<210> 18
<211> 25
<212> DNA
<213> artificial sequence

<220> synthetic DNA
<223> probe sequence of the U42627_at probe set of Affymetrix, the position of said probe in the mRNA sequence of the dusp6 gene being 1941

<400> 18
ttagctctgt atttgtggta tcggt 25

<210> 19
<211> 25
<212> DNA
<213> artificial sequence

<220> synthetic DNA
<223> probe sequence of the U42627_at probe set of Affymetrix, the position of said probe in the mRNA sequence of the dusp6 gene being 2007

<400> 19
aatatttgat cttcacttga gagtg 25

<210> 20
<211> 25
<212> DNA
<213> artificial sequence

<220> synthetic DNA
<223> probe sequence of the U42627_at probe set of Affymetrix, the position of said probe in the mRNA sequence of the dusp6 gene being 2013

<400> 20
tgatcttcac ttgagagtgt ttgtt 25

<210> 21
<211> 20
<212> DNA
<213> artificial sequence

<220> synthetic DNA
<223> direct primer designed to amplify, in combination with SEQ ID NO: 22, cDNA of the rat glyceraldehyde 3-phosphate dehydrogenase gene

<400> 21
aaggctgggg ctcacctgaa 20

<210> 22
<211> 21
<212> DNA
<213> artificial sequence

<220> synthetic DNA
<223> reverse primer designed to amplify, in combination with SEQ ID NO: 21, cDNA of the rat glyceraldehyde 3-phosphate dehydrogenase gene

<400> 22
ggcatggact gtggtcatga g 21

<210> 23
<211> 20
<212> DNA
<213> artificial sequence

<220> synthetic DNA
<223> a dusp6 gene antisense oligonucleotide

<400> 23
cgttgagcca cgccaccgtc 20

<210> 24
<211> 23
<212> DNA
<213> artificial sequence

<220> synthetic DNA
<223> primer used in quantitative real-time RT-PCR for evaluating the expression of dusp6 gene in post-mortem human tissue samples from optic nerve

<400> 24

```
cctgaggcca tttctttcat aga                                  23

<210> 25
<211> 23
<212> DNA
<213> artificial sequence

<220> synthetic DNA
<223> primer used in quantitative real-time RT-PCR for evaluating the expression
of dusp6 gene in post-mortem human tissue samples from optic nerve

<400> 25
gtcacagtga ctgagcggct aat                                  23
```

## Description

### FIELD OF THE INVENTION

The present invention refers to an *in vitro* method for detecting the presence of demyelinating diseases in an individual, for detecting the stage or severity of said diseases in the individual, or for monitoring the effect of the therapy administered to an individual presenting said diseases; to the search, identification, development and evaluation of efficacy of compounds for therapy of said diseases for the purpose of developing new medicaments; as well as to agents inhibiting the expression and/or activity of the DUSP6 protein, and/or to the effects of this expression. The methods and agents of the invention are preferably applied to multiple sclerosis.

### BACKGROUND OF THE INVENTION

Demyelinating diseases are those in which the main pathogenic process causes the destruction of the myelin sheath, which is necessary for the integrity of central nervous system cells. The main demyelinating diseases are: multiple sclerosis, Devic's syndrome, Baló disease, Marchiafava-Bignami disease, central pontine myelinolysis, acute disseminated encephalomyelitis, acute necrotizing hemorrhagic encephalomyelitis. Among them, multiple sclerosis is the most frequent disease due to alteration of the myelin in the central nervous system and, with the exception of trauma, it is the most frequent cause of neurological impairment in young adults. It is estimated that approximately 75% of the cases of demyelinating diseases correspond to multiple sclerosis.

Multiple sclerosis (MS) is the most frequent demyelinating disease of the central nervous system. It affects 1.5 million people worldwide, and its symptoms generally occur in young adults, therefore its consequences at a personal and socioeconomic level are very severe (Noseworthy et al. 2000 New Engl. J. Med., 343, 938-952).

Susceptibility to MS is due to unknown genetic and environmental factors. Prevalence of the disease is about 50 to 100 persons every 100,000 habitants in high risk regions, which are mainly located in northern areas of the northern hemisphere in Europe and America. The risk of suffering MS increases 10-20 fold in first degree relatives of patients, and concordance between monozygotic (genetically identical) twins increases up to 30-35%, whereas in dizygotic twins it only reaches 2-5% (Noseworthy et al. 2000 New Engl. J. Med., 343, 938-952). Genetic susceptibility is not characterized. To date, there is evidence that it can reside in some polymorphism of the genes encoding human leukocyte antigens (HLA), myelin oligodendrocyte glycoprotein (MOG) and other genes of chromosomes 10 and 15 (Steinman 2001, Nature Immunol 2, 762-764).

There is a consensus among MS researchers according to which the disease has two stages, an initial inflammatory phase of an autoimmune nature, and another secondary progressive neurodegenerative phase. In the first phase, activated T cells cross the hematoencephalic barrier, and once inside the central nervous system, they release proinflammatory cytokines triggering an immunological cascade ending in the destruction of the myelin and death of the oligodendrocytes. Knowledge of the autoimmune process with certain detail has served to develop agents of an immunomodulating nature, therapeutic efficacy of which is very modest. Until now, different targets for intervention during the inflammatory phase of MS (Zamvil and Steinman, 2003, Neuron 38, 685-688) have been disclosed. Among them are those which are focused on reducing inflammation of the nervous system initiated by the activation of the myelin-specific T cells, promoting autoimmunity particularly against components of the myelin, entering the central nervous tissue and releasing in it proinflammatory cytokines such as interferon-$\gamma$ and tumor-$\alpha$ necrosis factor. The immunomodulator interferon-1$\beta$, approved for the treatment of remitting-recurrent MS, also prevents cellular interactions leading to the penetration of activated T cells through the vascular endothelium. Other treatments in clinical trial phase are focused on neutralizing the activity of proinflammatory cytokines and/or to enhance anti-inflammatory ones. However, no medication has been generated which delays or stops the progression of the neurodegenerative phase of the disease which takes a course with progressive neurological degeneration, and which is characterized by the occurrence of severe demyelinating lesions in the white substance with massive oligodendrocyte loss, atrophy and severe axonal damage.

Glutamic acid is the main central nervous system exciter neurotransmitter. It activates a broad family of well characterized receptors at a molecular and functional level (Dingledine et al., 1999, Pharmacol. Rev. 51:7-61.), and it is removed form the extracellular space by means of carriers (Danbolt, 2001, Prog. Neurobiol 65, 1-105). Decrease of the efficacy of glutamic acid transport may cause the receptor overexcitation and trigger neuronal death. This phenomenon is relevant in the etiology of some neurological disorders, among which neurodegenerative diseases are included (Lee et al., 1999, Nature 399:A7-14).

Oligodendrocytes are the cells responsible for central nervous system myelinization, and their death causes severe alterations in nervous communication. Just like neurons, oligodendrocytes express glutamic acid receptors and transporters thereof, the molecular entity of which has been characterized in recent years. Likewise, it has been observed that overactivation of glutamatergic receptors causes oligodendroglial death. These results have led to postulate that glutamic acid homeostasis is fundamental for preventing possible oligodendroglial excitotoxicity, and that the latter may be a component in the etiopathogeny of demyelinating diseases (revised in Matute et al., 2001, Trends Neurosci. 24:224-230; Matute et al., 2002, Eur. J. Pharmacol, 447:239-246). Therefore, full understanding of the excitotoxic process may provide important data which, among other things, serve to establish possible therapeutic targets to act on by means of the development of new drugs.

Excitotoxicity can be mediated by glutamatergic receptors of the NMDA, AMPA or kainate type. Only the last two participate in oligodendrocytes, as these cells lack NMDA receptors (Sánchez-Gómez and Matute, 1999, Neurobiol. Dis. 6:475-485), and excitotoxic death of these cells is caused by an overload in cytosolic calcium levels (Alberdi et al., 2002, Neurobiol. Dis. 9:234-243). Excessive increase of this metabolite causes the activation of proteases, lipases and endonucleases, which can damage structural proteins, the membrane and DNA, respectively. In turn, excitotoxic cell death can occur by means of apoptosis or necrosis. However, the cellular mechanisms leading to either process, and the molecules intervening, are not well known.

The existence of *in vivo* experimental excitotoxic damage models allow examining the relevance of the findings obtained with the studies carried out in oligodendrocyte cultures. Thus, the infusion of excitotoxins in the optic nerve causes demyelination in plaques similar to MS lesions (Matute, 1998, Proc. Natl. Acad. Sci. USA 95:10229-10234). On the other hand, ischemic processes affecting the central nervous system cause massive excitotoxic damage to the oligodendroglial population (Li and Stys, 2000, J. Neurosci. 20:1190-1198). Finally, experimental autoimmune encephalomyelitis causes demyelination which can be largely reduced by means of the administration of glutamatergic receptor antagonists (Pitt et al., 2000, Nat. Med. 6:67-70; Smith et al., 2000, Nat. Med. 6:62-66). As a whole, experimental studies of demyelinating diseases in cellular and animal models indicate that excitotoxic death of oligodendrocytes is one component in the etiology of these diseases, and particularly of MS.

The DUSP (abbreviation for *dual specificity protein tyrosine phosphatases)* phosphatase family is involved in the regulation of kinases activated by extracellular signals (ERK), by cellular stress (JNK/SAPK) and by mitogens (MAPK). These kinases are activated by reversible phosphorylation of threonines and tyrosines and are inactivated by DUSPs which dephosphorylate these same moieties (Muda et al., 1996, J. Biol. Chem. 271:27205-27208). Just like the kinases that they regulate, DUSPs have been related with cell differentiation, regeneration and apoptosis. In relation to this last biological process, it has been observed that DUSP6 (also known as MKP-3, or MAP kinase phosphatase-3), (GeneBank code rat NM 053883, human NM_001946) has a cytosolic localization and induces apoptosis in endothelial cells in response to proinflammatory cytokines due to a mechanism which, in the last extreme, causes proteolysis of the anti-apoptotic Bcl-2 protein (Rossig et al., 2000, J. Biol. Chem. 275:25502-25507). However, the dusp6 gene, or the protein it encodes, DUSP6, has never been associated to apoptosis processes in nervous cells, or to demyelinating processes or diseases.

The authors of the present invention have discovered, after laborious research and using different techniques (DNA-chips and quantitative RT-PCR to measure gene expression levels, as well as inhibition assays of cell death due to apoptosis) that the expression of the dusp6 gene increases after excitotoxic stimuli in a pure oligodendrocyte culture, and that the blocking of its expression rescues these cells from death by apoptosis; and that the dusp6 gene expression is increased in post-mortem brain samples from human individuals affected with multiple sclerosis when compared to the expression in post-mortem brain samples from human individuals not affected with multiple sclerosis. These evidences convert dusp6 and the protein it encodes, DUSP6, into a useful target for the development of new *in vitro* methods of search, identification, development and evaluation of efficacy of compounds for therapy of demyelinating diseases, particularly for multiple sclerosis, for the purpose of developing new drugs.

Therefore, the present invention provides a high sensitivity *in vitro* method for detecting the presence of demyelinating diseases in an individual, for determining the stage or severity of said diseases in the individual, for monitoring the effect of the therapy administered to an individual presenting said diseases, or for deciding which patients suffering from demyelinating diseases are susceptible to being treated with drugs developed for inhibiting the expression and/or activity of the DUSP6 protein, and/or the effects of this expression The methods and agents of the invention are preferably applied to multiple sclerosis.

## OBJECT OF THE INVENTION

The main object of the present invention is the development of an *in vitro* method for detecting the presence of demyelinating diseases, for determining the stage or severity of said diseases in the individual, or for monitoring the effect of the therapy administered to an individual presenting said diseases.

A second object of the present invention is an *in vitro* method for searching for, identifying, developing and evaluating efficacy of compounds for the therapy of demyelinating diseases.

An additional object of the invention is based on the use of sequences derived from the dusp6 gene for the *in vitro* diagnosis and prognosis of demyelinating diseases; as well as for the search, identification, development and evaluation of efficacy of compounds for the therapy of said diseases.

Another object of the present invention consists of providing agents characterized in that they inhibit the expression and/or activity of the DUSP6 protein for the treatment of demyelinating diseases.

Lastly, also object of the invention is a pharmaceutical composition comprising one or several therapeutic agents together with a pharmaceutically acceptable excipient for the treatment of demyelinating diseases.

## DESCRIPTION OF THE FIGURES

Figure 1 shows a melting curve of the PCR product of reference gene, GADPH (Tm = 85 °C) (figure 1a) and of the target gene, dusp6 (Tm = 77 °C) (figure 1b), in measurement experiments of gene expression by real time quantitative RT-PCR. Temperature (°C) is represented on the axis of abscissas and fluorescence (d(F1)/dT) is represented on the axis of ordinates.

Figure 2 shows the calculation of the amplification efficacy of the dusp6 PCR reactions in gene expression measurement experiments by real time quantitative RT-PCR. The standard line obtained for dusp6 data. The concentration logarithm is shown on the axis of abscissas and the crossing point is shown on the axis of ordinates.

Figure 3 shows the effect of antisense oligonucleotides against caspase-3 (figure 3a) and against dusp6 (figure 3b) on the oligodendroglial death process after stimulation with AMPA 10 μM + CTZ 100 μM for 15 min. ** p<0.01, *p<0.05, n=3-5.

Figure 4 shows the alignment of human (NM_001946) and rat (NM_053883) dusp6 nucleotide sequences.

Figure 5 shows the alignment of human (NP_001937) and rat (NP_446335) dusp6 amino acid sequences.

Figure 6 shows Dusp6 gene expression levels in postmortem human optic nerve samples. MS means multiple sclerosis.

## DETAILED DESCRIPTION OF THE INVENTION

To facilitate comprehension of the present patent application, the meaning of some terms and expressions in the context of the invention will be explained below:

The terms "subject" or "individual" refer to members of mammal species, and includes, but is not limited to, domestic animals, primates and humans; the subject is preferably a human being, male or female, of any age or race.

The expression "demyelinating diseases" refers to those diseases in which the main pathogenic process causes the destruction of the myelin, which is the lipoprotein layer covering nerves and facilitates the transmission of impulses through nervous fibers. In the scope of the present invention, demyelinating diseases include: multiple sclerosis, Devic's syndrome, Baló disease, Marchiafava-Bignami disease, central pontine myelinolysis, acute disseminated encephalomyelitis, acute necrotizing hemorrhagic encephalomyelitis.

The term "gene" refers to a molecular chain of deoxyribonucleotides encoding a protein.

The term "DNA" refers to deoxyribonucleic acid. A DNA sequence is a deoxyribonucleotide sequence.

The term "cDNA" refers to a nucleotide sequence complementary to an mRNA sequence.

The term "RNA" refers to ribonucleic acid. An RNA sequence is a ribonucleotide sequence.

The term "mRNA" refers to messenger ribonucleic acid, which is the fraction of total RNA translated into proteins.

The phrase "mRNA transcribed from" refers to transcription of the gene (DNA) into mRNA as a first step for the gene to be expressed and translated into a protein.

The term "nucleotide sequence" indistinctively refers to a ribonucleotide (RNA) or deoxyribonucleotide (DNA) sequence.

The term "protein" refers to a molecular chain of amino acids bonded by covalent or non-covalent bonds. The term includes all the post-translation modification forms, for example, glycosylation, phosphorylation or acetylation.

The terms "peptide" and "polypeptide" refer to molecular chains of amino acids representing a protein fragment. The terms "protein" and "peptide" are used indistinctively.

The term "antibody" refers to a glycoprotein exhibiting a specific binding activity for a particular protein, which is called "antigen". The term "antibody" comprises monoclonal antibodies, or polyclonal antibodies, intact or fragments of them; and it includes human, humanized and non-human origin antibodies. "Monoclonal antibodies" are homogenous populations of highly specific antibodies directed against a single site or antigen "determinant". "Polyclonal antibodies" include heterogeneous populations of antibodies directed against different antigen determinants.

The term "epitope", as it is used in the present invention, refers to an antigen determinant of a protein, which is the amino acid sequence of the protein which a specific antibody recognizes.

The term "solid phase", as it is used in the present invention, refers to a non-aqueous matrix which the antibody can bind to. Examples of solid phase materials include glass, polysaccharides, for example agarose, polyacrylamide, polystyrene, polyvinyl alcohol and silicones. Examples of solid phase forms are an assay plate well or purification column.

The term "oligonucleotide primer", as it is used in the present invention, refers to a nucleotide sequence, which is complementary of a dusp6 gene nucleotide sequence. Each primer hybridizes with its target nucleotide sequence and acts as a DNA polymerization start site.

The term "probe", as it is used in the present invention, refers to a complementary nucleotide sequence of a dusp6 gene nucleotide sequence, which can be used to detect that nucleotide sequence derived from the dusp6 gene.

The term "therapeutic target" refers to nucleotide or peptide sequences against which a drug or therapeutic compound can be designed and clinically applied.

The term "antagonist" refers to any molecule inhibiting the biological activity of the antagonized molecule. Examples of antagonist molecules include, among others, proteins, peptides, natural peptide sequence variations and small organic molecules (having a molecular weight of less than 500 Daltons).

The present invention is based on the discovery that both dusp6 gene expression and the DUSP6 protein concentration are increased during cell death of oligodendrocytes, which are the cells responsible for central nervous system myelinization; on the discovery that blocking dusp6 expression with a antisense RNA specific of this gene blocks the death process of oligodendrocytes, therefore blocking the demyelinization process; and on the discovery that dusp6 gene expression is increased in post-mortem brain samples from human individuals affected with multiple sclerosis when comparing the expression in post-mortem brain samples form human individuals not affected with multiple sclerosis.

In this sense, the present invention, in the first place, provides an in vitro method for detecting the presence of demyelinating diseases in an individual, for determining the stage or severity of said diseases in the individual, or for monitoring the effect of the therapy administered to an individual presenting said diseases, comprising:

a) detection and/or quantification of the DUSP6 protein, of the dusp6 gene mRNA or the corresponding cDNA in a sample from said individual, and

b) comparison of the DUSP6 protein amount, of the dusp6 gene mRNA amount, or of the corresponding cDNA amount detected in a sample from an individual, with the DUSP6 protein amount, with dusp6 gene mRNA amount, or with the corresponding cDNA amount detected in the samples of control individuals, or in previous samples from the same individual, or with normal reference values.

The method provided by the present invention is of a high sensitivity and specificity, and is based on the fact that subjects or individuals diagnosed with a demyelinating disease, especially multiple sclerosis, present high levels of mRNA transcribed from the dusp6 gene (*high dusp6 gene expression levels*), or high concentrations of the protein encoded by the dusp6 gene (DUSP6 *protein*), in comparison with corresponding levels in samples from subjects with no clinical history of demyelinating diseases.

The present method comprises a step for obtaining the sample from the individual. It is possible to work with different fluid samples such as, for example: serum, urine, saliva, feces or cerebrospinal fluid. The sample can be obtained by any conventional method, preferably by surgical resection.

The samples can be obtained from subjects previously diagnosed, or not diagnosed, with a demyelinating disease, preferably multiple sclerosis; or also from a subject undergoing treatment, or who has been previously treated for a demyelinating disease, particularly for multiple sclerosis.

The present method also comprises a sample extraction step, either for obtaining the protein extract from the latter, or for obtaining the total RNA extract. One of these two extracts represents the working material for the following phase. Total protein or total RNA extraction protocols are well known by a person skilled in the art (Chomczynski P. et al., Anal. Biochem., 1987, 162: 156; Chomczynski P., Biotechniques, 1993, 15: 532).

Any conventional test can be used within the framework of the invention for detecting a demyelinating disease, as long as it measures *in vitro* levels of mRNA transcribed from the dusp6 gene or its complementary cDNA, or the DUSP6 protein concentration, in samples taken from the individuals to be analyzed and from control individuals.

Therefore, this invention provides a method for detecting the presence of demyelinating diseases, especially multiple sclerosis, in an individual, for determining the stage or severity of said diseases, or for monitoring the effect of the therapy administered to an individual presenting said diseases, either based on the DUSP6 protein concentration, or on the measurement of the dusp6 gene expression level.

In the event that the DUSP6 protein is to be detected, the method of the invention comprises a first step of contacting the protein extract of the sample with a composition of one or more specific antibodies against one or more epitopes of the DUSP6 protein, and a second step for quantifying the complexes formed by antibodies and the DUSP6 protein.

There is a wide variety of immunological assays available for detecting and quantifying the formation of specific antigen-antibody complexes; numerous competitive and non-competitive protein binding assays have previously been disclosed, and a large number of these assays are commercially available.

Thus, the DUSP6 protein can be quantified with antibodies such as, for example: monoclonal antibodies, polyclonal antibodies, intact or recombinant fragments thereof, "combibodies" and Fab or scFv antibody fragments, specific against the DUSP6 protein; these antibodies being human, humanized or of non-human origin. The antibodies used in these assays can be labeled or not; the unlabeled antibodies can be used in agglutination assays; the labeled antibodies can be used in a wide variety of assays. Marker molecules which can be used to label antibodies include radionucleotides, enzymes, fluorophores, chemoluminescent reagents, enzyme substrates or cofactors, enzyme inhibitors, particles, colorants and derivatives.

There is a wide range of well known assays which can be used in the present invention using unlabeled antibodies (primary antibody) and labeled antibodies (secondary antibody); included among these techniques are the Western-blot or Western transfer, ELISA (Enzyme-Linked Immunosorbent Assay), RIA (Radioimmunoassay), competitive EIA (Competitive Enzyme Immunoassay), DAS-ELISA (Double Antibody Sandwich-ELISA), immunocytochemical or immunohistochemical techniques, techniques based on the use of biochips or microarrays of proteins including specific antibodies, or assays based on colloidal precipitation in formats such as *dipsticks.* Other ways to detect and quantify the DUSP6 protein include affinity chromatography techniques, ligand binding assays or lectin binding assays.

The immunoassay preferred in the method of the invention is a double antibody sandwich-ELISA (*DAS-ELISA*) assay. In this immunoassay, any antibody or combination of antibodies specific against one or more epitopes of the DUSP6 protein can be used. As an example of one of the many possible formats of this assay, a monoclonal or polyclonal antibody, or a fragment of this antibody, or a combination of antibodies coating a solid phase, are put into contact with the sample to be analyzed, and incubated for a certain time and under conditions suitable for forming antigen-antibody complexes. After a rinsing under conditions suitable for removing the non-specific complexes, an indicator reagent, comprising a monoclonal or polyclonal antibody, or a fragment of this antibody, or a combination of these antibodies, bonded to a signal-generating compound, is incubated with the antigen-antibody complexes under suitable conditions and time. The presence of the DUSP6 protein in the sample to analyze is detected and quantified, if any, by measuring the generated signal. The DUSP6 protein amount present in the sample to analyze is proportional to that signal.

In the event that the mRNA or cDNA corresponding to the dusp6 gene, and not the protein, is to be detected, the method of the invention for detecting *in vitro* a demyelinating disease has different steps. Thus, once the sample is obtained and the total RNA is extracted, according to the method of the invention, detection of the mRNA or corresponding cDNA of the dusp6 gene is carried out, comprising a first amplification step of the total RNA extract or corresponding cDNA synthesized by mRNA reverse transcription, and a second quantification step of the amplification product of the mRNA or cDNA of the dusp6 gene.

One example of mRNA amplification consists of reverse transcription mRNA into cDNA (RT), followed by Polymerase Chain Reaction (PCR) using oligonucleotide primers, the sequences of the primers used being SEQ ID NO:1 and SEQ ID NO:2; PCR is a technique for amplification of a certain nucleotide sequence (target) contained in a nucleotide sequence mixture. In PCR, an oligonucleotide primer pair excess is used, which primers hybridize with the target nucleotide sequence complementary strands. Then, an enzyme with polymerase activity (DNA Taq Polimerase) extends each primer, using the target nucleotide sequence as a template. The extension products are then converted into target sequences after the disassociation of the original target strand. New primer molecules hybridize and the polymerase extends them; the cycle is repeated to exponentially increase the number of target sequences. This technique is disclosed in US patent 4,683,195 and US patent 4,683,202. Different methods have previously been disclosed for detecting and quantifying PCR amplification products, any of which can be used in this invention. In a preferred method of the invention, the amplified product is detected by agarose gel electrophoresis in the following manner: five microliters of the amplification product are subjected to separation by agarose gel electrophoresis at a 2% concentration in a TAE 1x buffer at 100 Volts for one hour. After electrophoresis, the gel is stained with ethidium bromide, and the amplification product is visualized when illuminating the gel with ultraviolet (uv) light; as an alternative to staining, and a preferred embodiment, the amplified product can be transferred to a nylon membrane by *Southern blotting or Southern transfer* techniques to be detected with a suitably marked, specific probe of the dusp6 gene cDNA.

In another example, mRNA detection is carried out by transferring the mRNA to a nylon membrane by means of transfer techniques such as, for example, Northern-blot or Northern transfer, and detecting it with specific probes of the mRNA or the corresponding cDNA of the dusp6 gene.

In a particular embodiment, amplification and quantification of the mRNA corresponding to the dusp6 gene is carried out at the same time by means of real time quantitative RT-PCR (Q-PCR).

The final step of the method of the invention for detecting in vitro a demyelinating disease in a sample from an individual comprises comparing the DUSP6 protein amount, the dusp6 gene mRNA amount, or the corresponding cDNA amount detected in a sample from an individual, with the DUSP6 protein amount, the dusp6 gene mRNA amount, or the corresponding cDNA amount detected in control subject samples or with normal reference values.

As its second object, the invention provides an *in vitro* method for identifying and evaluating the efficacy of agents for therapy of demyelinating diseases, especially multiple sclerosis:

a) treating rat optic nerve oligodendrocyte primary culture with stimuli relevant to demyelinating diseases, preferably with excitotoxic stimuli such as Ampa or Kainate,

b) detecting and quantifying changes in the dusp6 gene or DUSP6 protein expression in cells of the culture in response to said stimuli,

c) putting the stimulated primary oligodendrocyte culture obtained in step a) into contact with the candidate compound under the conditions and for the time suitable for permitting them to interact,

d) detecting and quantifying the dusp6 gene or DUSP6 protein expression levels, and

e) comparing the expression levels obtained in step d) with the corresponding levels in pure stimulated oligodendrocyte cultures not treated with the candidate compound.

Quantification of the dusp6 gene or DUSP6 protein expression levels is carried out in a manner similar to how it is indicated in the method of the invention for detecting *in vitro* the presence of a demyelinating disease in an individual.

When an agent decreases dusp6 gene expression levels or reverts the effects of the increased expression of said gene, preferably blocking the cell death process, this agent is converted into a candidate for the therapy of demyelinating diseases, especially multiple sclerosis.

Another aspect of the invention refers to the use of nucleotide or peptide sequences derived form the dusp6 gene in methods of search, identification, development and evaluation of efficacy of compounds for therapy of demyelinating diseases, especially multiple sclerosis. Within the search methods, the importance acquired recently by drug screening methods, based on the binding, competitive or not, of the potential drug molecule to the therapeutic target, stands out.

Another additional object of the invention refers to the use of nucleotide or peptide sequences derived from the dusp6 gene for detecting the presence of demyelinating diseases, especially multiple sclerosis, for determining the stage or severity of said diseases in the individual, or for monitoring the effect of the therapy administered to an individual presenting said diseases.

Another object of the invention consists of providing agents characterized in that they inhibit DUSP6 protein expression and/or activity. These agents, which can be identified and evaluated according to the present invention, can be chosen from the group formed by:

a) an antibody, or combination of antibodies, specific against one or more epitopes present in the DUSP6 protein, preferably a human or humanized monoclonal antibody; also being possible a fragment of the antibody, a simple-chain antibody or an anti-idiotype antibody,

b) cytotoxic agents, such as toxins, molecules with radioactive atoms, or chemotherapeutic agents, which include, without limitation, small organic and inorganic molecules, peptides, phosphopeptides, antisense molecules, ribozymes, siRNAs, triple helix molecules, etc., inhibiting DUSP6 protein expression and/or activity, such as, for example, the dusp6 specific antisense oligonucleotides SEQ ID NO:3 and SEQ ID NO:4, which inhibit cell death of oligodendrocytes treated with AMPA, or any antisense oligonucleotide with an homology with said molecule exceeding 50%, or any dusp6 specific antisense oligonucleotide inhibiting its expression, and

c) DUSP6 protein antagonist compounds inhibiting one or more of the DUSP6 protein functions.

Lastly, a pharmaceutical composition comprising a therapeutically effective amount of one or several agents of those previously mentioned, together with one or more excipients and/or carrier substances also constitutes an object of the present invention. Furthermore, said composition may contain any other active ingredient inhibiting DUSP6 protein function.

The excipients, carrier substances and auxiliary substances must be pharmaceutically and pharmacologically tolerable, such that they can be combined with other components of the formulation or preparation and do not exercise adverse effects on the treated organism. Pharmaceutical compositions or formulations include those which are suitable for oral or parenteral administration (including subcutaneous, intradermal, intramuscular and intravenous), although the best administration route depends on the patient's condition. The formulations can be in single dose form. The formulations are prepared according to methods known in the field of pharmacology. The amounts of active substances to be administered can vary according to the particularities of therapy.

The following examples illustrate the invention.

## EXAMPLES

### Example 1. - Differential analysis of dusp6 gene expression in oligodendrocyte samples using the microarrays *Rat Genome U34 DNA arrays.*

### 1.1. Materials and methods

Microarrays. The microarrays *GeneChip Test 3* (Affymetrix, Santa Clara) were used which allow testing the quality of the RNA prior to the expression analysis with *GeneChip Rat Genome U34 set* (Affymetrix, Santa Clara), representing 3322 complete gene sequences. The dusp6 gene is represented in the microarray by probe set U42627_at of Affymetrix, which are *sense* oligonucleotides of 25 nucleotides in length designed on the basis of GeneBank U42627 sequence (Table 1).

Table 1.

| Description of probes corresponding to U42627_at probe set. | | |
|---|---|---|
| **Consecutive order of probes** | **Probe sequence (5'-3')** | **Position of probe in mRNA sequence of gene** |
| 1 | SEQ ID NO:5 | 1557 |
| 2 | SEQ ID NO:6 | 1563 |
| 3 | SEQ ID NO:7 | 1623 |
| 4 | SEQ ID NO:8 | 1713 |
| 5 | SEQ ID NO:9 | 1725 |
| 6 | SEQ ID NO:10 | 1773 |
| 7 | SEQ ID NO:11 | 1827 |
| 8 | SEQ ID NO:12 | 1845 |
| 9 | SEQ ID NO:13 | 1857 |
| 10 | SEQ ID NO:14 | 1863 |
| 11 | SEQ ID NO:15 | 1881 |
| 12 | SEQ ID NO:16 | 1887 |
| 13 | SEQ ID NO:17 | 1935 |
| 14 | SEQ ID NO:18 | 1941 |
| 15 | SEQ ID NO:19 | 2007 |
| 16 | SEQ ID NO:20 | 2013 |

**Samples**. The samples studied corresponded to primary oligodendrocyte cultures obtained from the optic nerve of 12-day old rats. To prevent inter-individual variations, each culture corresponded to a 10-animal cell pool. Treatments were made in duplicate with 10 μM AMPA (α-amino-3-hydroxy-5-methyl-4-isoxazolepropionic acid) or 3μM kainate. The duration of treatments was 2, 10 and 15 minutes, the cells being collected immediately after treatment in the first two cases and 1 hour after the 15-minute treatment. Treatment for 10 and 15 minutes with AMPA and with kainate causes apoptosis, whereas 2-minute treatment was considered sublethal due to scarce treatment duration. After treatments, the cells were collected in TRIzol® Reagent (Life Technologies) and were stored at -80°C until total RNA extraction.

### *Gene*Chip gene expression analysis

The analysis was carried out with total RNA from oligodendrocyte cultures. Each sample corresponded to the mixture of total RNAs from two cultures and were called the following:

- control: untreated cultured oligodendrocytes
- ampa 2: oligodendrocytes treated for 2 minutes with 10 μM AMPA and collected after treatment

- ampa 10: oligodendrocytes treated for 10 minutes with 10 μM AMPA collected after treatment
- ampa 15: oligodendrocytes treated for 15 minutes with 10 μM AMPA and collected 60 minutes later
- Kainate 2: oligodendrocytes treated for 2 minutes with 3 μM kainate and collected after treatment
- Kainate 10: oligodendrocytes treated for 10 minutes with 3 μM kainate and collected after treatment
- Kainate 15: oligodendrocytes treated for 15 minutes with 3 μM kainate and collected 60 minutes later

**cRNA synthesis**

The total RNA of each one of the cultures was obtained by homogenizing the cells in TRIzol® Reagent (Life Technologies) following the supplier's recommendations. The resulting total RNA was cleaned with the RNeasy Mini kit (QIAGEN) (Chomczynski P. et al., Anal. Biochem., 1987, 162: 156; Chomczynski P., Biotechniques, 1993, 15: 532). 2 μg from each total RNA preparation were used as starting material for the synthesis of biotinylated cRNA with the MessageAmp™ aRNA kit (Ambion).

**Array hybridization and scanning**

15 μg of each biotinylated cRNA were fragmented at 94°C for 35 minutes in a buffer solution containing 40 mM Tris-Acetate (pH 8.1), 100 mM KOAc and 30 mM MgOAc. The fragmented cRNA was mixed with hybridization buffer (100mM MES, 1M NaCl, 20 mM EDTA, 0.01% Tween 20) and was heated at 99°C for 5 minutes and subsequently at 45°C for 5 minutes, to then be loaded into the Affymetrix array. The first array in which hybridization was carried out was Affymetrix Test 3. This array allows testing the quality of the RNA prior to expression analysis in the Affymetrix® GeneChip® Rat Genome U34 set (RG-U34).

For hybridization, the arrays were incubated in a rotary oven at 45°C for 16 hours and with a constant rotation of 60 rpm.

Washing and staining of each array was carried out in the Affymetrix® Fluidics Station. A washing and staining program was used which included:

- 10x2 washing cycles with SSPE-T 6x (0.9 M NaCl, 60 mM $NaH_2PO4$, 6 mM EDTA, 0.01% Tween 20) at 25°C,
- 4x15 cycles with 0.1 mM MES, 0.1M NaCl, 0.01% Tween 20 at 50°C,
- staining of biotinylated cRNA with a streptavidin-phycoerythrin conjugate (10 μg/ml Molecular Probes)
- 10x4 washing cycles with SSPE-T at 25°C
- staining with an anti-streptavidin antibody (3 μg/ml, Vector laboratories) for 10 minutes
- staining with a streptavidin-phycoerythrin conjugate (10 μg/ml, Molecular Probes) for 10 minutes
- 15x4 washing cycles with SSPE-T at 30°C

The arrays were scanned at 560 nm using a confocal microscope using laser emission (Agilent GeneArray Scanner). Analysis of the intensity readings was carried out with Microarray Suite 5.0 software. To compare the arrays, the latter were scaled to a total intensity of 100.

**1.2. Results.**

Differential analysis of the dusp6 gene expression in the oligodendrocytes stimulated with regard to the control was carried out from the array comparison data obtained using the Affymetrix software. The parameters taken into account (in the order in which they appear in the list) were: i) Detection. This indicates if the transcribed element is Present (P), Absent (A) or Marginal (M), ii) Change: This indicates if the expression of a certain transcribed element Increases (I), Decreases (D), undergoes No Change (NC), Marginally Increases (MI), or Marginally Decreases (MD), iii) Signal Log Ratio (SLR): This indicates the level of expression change between the baseline (control) and a test sample. This change is expressed as $\log_2$ of the ratio *(fold change* or number of times the expression of the gene is increased or inhibited in the treated test sample in comparison to the untreated control sample). An SLR value of 1 (equivalent to a *fold change* of 2) is considered significant for transcribed elements the expression of which increases in comparison to the control, and an SLR value of -1 is considered significant for transcribed elements the expression of which decreases in comparison to the control.

Table 2.

| Results obtained with the Rat Genome U34 array for dusp6, Genebank accession number U42627. | | | | | |
|---|---|---|---|---|---|
| | Signal | Detection | SLR (vs control) | Ratio (=$2^{SLR}$) | Change |
| Control | 34.1 | P | | | |
| AMPA 2 | 75.3 | P | 0.8 | 1.7 | I |
| AMPA 10 | 101.7 | P | 1.2 | 2.3 | I |
| AMPA 15 | 149.7 | P | 2.1 | 4.3 | I |
| Kainate 2 | 48.4 | P | 0.6 | 1.5 | I |
| Kainate 10 | 43.5 | P | 0.6 | 1.5 | I |
| Kainate 15 | 63.7 | P | 0.9 | 1.9 | I |

**1.3. Discussion**

Differential analysis of the dusp6 gene expression in oligodendrocyte cultures treated with en AMPA with regard to the control showed that dusp6 gene expression levels were increased more than 4 fold (SLR=2.1) in the 15-minute treatment and more than 2 fold (SLR=1.2) in the 10-minute treatment, whereas the expression increase was less than two fold (SLR<1) in treatments with kainate.

**Example 2.- Differential analysis of DUSP6 protein expression in oligodendrocyte cultures, using the real time quantitative RT-PCR technique.**

**2.1. Materials and methods.**

The method used consists of reverse transcription of mRNA to cDNA and its subsequent amplification into a *LightCycler* equipment*(Roche)*, using SYBR Green for the detection of the amplified product. Real time quantification is carried out, and it allows calculating the relative expression of the sequence in different samples in the linear amplification phase of the reaction.

**Samples**: Primary oligodendrocyte culture samples treated with AMPA were analyzed. The samples came from cultures different from those analyzed with DNA microarrays. 4 cultures were carried out:

- control: untreated cultured oligodendrocytes
- ampa 2: oligodendrocytes treated for 2 minutes with 10 μM AMPA and collected after treatment
- ampa 10: oligodendrocytes treated for 10 minutes with 10 μM AMPA collected after treatment
- ampa 15: oligodendrocytes treated for 15 minutes with 10 μM AMPA and collected 60 minutes later.
- kainate 2: oligodendrocytes treated for 2 minutes with 3 μM kainate and collected after treatment
- kainate 10: oligodendrocytes treated for 10 minutes with 3 μM kainate and collected after treatment
- kainate 15: oligodendrocytes treated for 15 minutes with 3 μM kainate and collected 60 minutes later.

The cells were collected in TRIzol® Reagent (Life Technologies) and were stored at -80°C until the moment of total RNA extraction.

**Real time quantitative RT-PCR.** The total RNA of each one of the cultures was obtained by homogenizing the cells in TRIzol® Reagent (Life Technologies) following the supplier's recommendations. The resulting total RNA was cleaned with the RNeasy kit (QIAGEN) (Chomczynski P. et al., Anal. Biochem., 1987, 162: 156; Chomczynski P., Biotechniques, 1993, 15: 532). The total RNA samples were conserved at -80°C from the moment they were obtained until they were used. The RNA was spectrophotometrically quantified and 5 μg of total RNA were digested with DNase (2 U/μl) of Ambion at a concentration of 2U per μg of RNA, for 30 minutes at 37°C.

**cDNA synthesis**

1 μg of RNA treated with DNAse as a starting material was used for the synthesis of the first cDNA strand with the reverse transcriptase enzyme SuperScript™ II RNase (Life Technologies), using an oligo-dT oligonucleotide as a primer which contained the sequence of the promoter of the phage T7 RNA polymerase. Aliquots of cDNA diluted to the working concentration were used.

### Amplification

Synthesized cDNA was amplified using specific primers of the rat dusp6 gene (SEQ ID NO:1 and SEQ ID NO:2), and of the gene encoding the rat protein glyceraldehyde 3-phosphate dehydrogenase, Genebank accession number NM_017008 (SEQ ID NO:21 and SEQ ID NO:22). Real time PCR reactions were prepared using the LightCycler-FastStart DNA master SYBR Green I kit (Roche) following the manufacturer's instructions. The amplification program consisted of 1 95°C cycle for 10 min ("hot start") followed by 45 95°C cycles (denaturation) for 10 seconds, 60°C (annealing) for 5 seconds, 72°C (amplification and fluorescence acquisition) for 10 seconds. The melting curve analysis program consisted of a one-pulse cycle of 95°C, 65°C for 15 seconds, and a 95°C pulse during the amplification and acquisition step.

### Quantification

First, specificity of the PCR products was determined by analyzing the melting curves. Subsequently, as a relative measurement of gene expression, the ratio between the abundance of dusp6 transcribed mRNAs and the abundance of GAPDH transcribed mRNAS was calculated, and the data of the ratio was standardized in each one of the treated samples on the basis of control sample values. To calculate efficiency of the PCR reactions (dusp6 and GAPDH), a standard curve was constructed for each gene sequence carried out with cDNA serial dilutions. The template cDNA concentrations for the reactions on the standard curve were arbitrarily given the values 2x, 0.5x, 0.2x and 0.02x. The crossing point values for each one of the reactions were used to construct the standard curve: y=mx+b, where m is the slope and b is the intersection point of the line on the axis of ordinates. Starting RNA concentration values in each sample were obtained from the data of the line using the following formula:

$$[RNA]= 10^{(Cp-b)/m}$$

where Cp is the crossing point.

The units of the calculated concentration are the same as those used for constructing the standard curve, therefore in this case they are arbitrary units disappearing in subsequent calculations as they are ratios.

The concentration values obtained for dusp6 in each sample were standardized in comparison to the values obtained for GAPDH (used in this sense as a "housekeeping" gene, the expression of which does not vary as a consequence of treatment).

The change rate at each point was obtained by dividing the concentration values standardized in comparison to the concentration values in the control.

### 2.2. Results.

The real time quantitative RT-PCR results were used to obtain the dusp6 gene expression change rate in treated oligodendrocyte samples with regard to the untreated control.

### Melting curve analysis

PCR product analysis showed the specific amplification of a product with a melting temperature similar to that estimated according to the software used for primer design, *PrimerExpress (Applied Biosystems)* (Figure 1).

### Calculation of efficiency of PCR reactions

Two replicas of each one of the 4 cDNA dilutions were amplified, and the cutting points (Cp) of each one of them were represented in a graph with regard to the logarithm of the cDNA concentration to construct the standard line (Table 3 and figure 2).

Table 3.

| Crossing points (*Cp*) of the amplification of the 4 cDNA dilutions for each replica amplified with the dusp6 gene primers. | | |
|---|---|---|
| [RNA] | Log [RNA] | Cp |
| 2x | 0.30103 | 25.91 |
| 2x | 0.30103 | 26.04 |
| 0.5x | -0.30103 | 27.88 |
| 0.5x | -0.30103 | 27.88 |
| 0.2x | -0.69897 | 29.54 |
| 0.2x | -0.69897 | 29.72 |
| 0.02x | -1.69897 | 32.90 |
| 0.02x | -1.69897 | 32.95 |

**Quantification of the dusp6 gene expression change**

Two replicas of each sample (control, AMPA2, AMPA10 and AMPA15) were amplified with the dusp6 and GAPDH specific primers. Expression changes of the dusp6 gene in the treated samples with regard to the untreated control (table 5) were calculated from the cutting points generated in these amplifications (table 4).

Table 4.

| Experimental dusp6 crossing points in the 4 samples | | | | |
|---|---|---|---|---|
| Replicas | Control | AMPA2 | AMPA10 | AMPA15 |
| 1 | 31.94 | 30.46 | 32.15 | 29.73 |
| 2 | 31.65 | 30.54 | 32.31 | 29.61 |
| *Mean* | *31.795* | *30.5* | *32.23* | *29.67* |
| *SD* | *0.205* | *0.056* | *0.113* | *0.084* |

Table 5.

| Dusp6 overexpression values (Fold Change) in the 3 treated samples with regard to the untreated control | |
|---|---|
| Sample | Ratio |
| AMPA 2 | 1.7 |
| AMPA 10 | 0.9 |
| AMPA 15 | 2.7 |

**2.3. Discussion.**

The results confirmed the data obtained in differential gene expression analysis with DNA-chips (example 1), thus, dusp6 expression was increased 2.7 fold in treatment with AMPA15. The different degree of sensitivity of the two techniques could explain the lack of concordance between results of the quantitative RT-PCR and of the DNA-chip for the AMPA10 sample in which a slight overexpression was observed in example 1.

**Example 3. Cell death inhibition in oligodendrocytes treated with AMPA after incubation with antisense oligonucleotides against the dusp6 gene.**

**3.1. Materials and methods.**

To evaluate if dusp6 expression blocking inhibits cell death of oligodendrocytes treated with AMPA, antisense oligonucleotides (ODNs) capable of blocking said gene expression were used.

Oligonucleotide design was carried out based on the secondary structure of the RNA, trying to prevent sequences presenting a high internal hybridization percentage, and with the aid of AO predict (http://www.cgb.ki.se/AOpredict/) computer software. To improve efficacy and capability to degrade RNA and to reduce side effects, last-generation oligonucleotides were designed, presenting two types of modifications; 2'-O-Methyl groups in the 6 first and last bases, and phosphorothiodated groups in the 8 intermediate bases. Furthermore, the oligonucleotides were labeled with Texas Red to be able to identify the cells transfected with said oligonucleotides.

Three antisense ODNs, for the purpose of identifying active sequences, plus a sense ODN, used as a negative control, were designed. To improve penetration thereof, the ODNs were transfected using the Lipofectin kit (Invitrogen).

Table with the antisense oligonucleotide sequences

ODN1- SEQ ID NO:23
ODN2- SEQ ID NO:3
ODN3- SEQ ID NO:4

24 hours after seeding, the oligodendrocytes were treated with a mixture of ODNs at different concentrations coupled to the transfection kit liposomes.

After 24 hours, the cells were rinsed and were left another 24 hours before the experiment to ensure total blocking of the protein under study. Subsequently, the excitotoxic stimulus, AMPA 10 μM + CTZ 100 μM, was added during 15 minutes, and the protecting effect of the different ODNs after 6 hours was assessed.

**3.2. Results.**

ODN transfection percentages of about 50% were observed. To complete the technique, antisense ODNs were designed against caspase-3, mediator of oligodendroglial death process induced by AMPA 10 μM + CTZ 100 μM. From among the 3 ODNs designed against caspase-3, ODN 2 achieved blocking the effect of the excitotoxic stimulus, showing validity of the method (figure 3A).

Then, antisense ODNs were designed against dusp6, and oligodendrocyte cultures were transfected according to that previously described. Thus, oligodendroglial death after stimulation with AMPA 10 μM + CTZ 100 μM was significantly inhibited in the presence of the antisense ODNs 2 ($p<0.01$; $n=5$) and 3 ($p<0.05$; $n=3$) against dusp6 (figure 3B).

**3.3. Discussion.**

The assays carried out showed that dusp6 contributes to the oligodendroglial death process, since blocking of the expression of said gene inhibited cell death induced by treatment with AMPA.

The results also suggested that the dusp6 gene specific antisense oligonucleotide, which inhibited cell death in oligodendrocytes treated with AMPA after incubation, could be a pharmacologically active molecule in treatment of demyelinating diseases, preferably multiple sclerosis.

**Example 4.- Comparison analysis of rat and human Dusp6 sequences.**

**4.1. Materials and methods**

For the purpose of comparing the degree of homology of the Dusp6 gene human and rat sequences, a nucleotide sequence alignment was carried out. The rat Dusp6 sequence was compared to the human Dusp6 sequence. The nucleotide sequences as well as the amino acid sequences were compared.

The nucleotide sequences to compare were:

- Rat Dusp6, GenBank accession number NM_053883;
- Human Dusp6, GeneBank accession number NM_001946.

The amino acid sequences to compare were:

- Rat Dusp6, GenBank accession number NP_446335;
- Human Dusp6, GeneBank accession number NP_001937.

Alignment was carried out using computer tools available on web page http://biobug.life.nthu.edu.tw/~tswang/pw-fasta/ .

### 4.2. Results

Results of the nucleotide and amino acid alignments are shown in figures 4 and 5, respectively.

### 4.3. Discussion

Alignment of the NM_001946 (human dusp6) and NM_053883 (rat dusp6) sequences revealed an 86.713% degree of identity on a sequence overlap of 1716 nucleotides.
With regard to the amino acid sequence comparison, the degree of identity between the NP_001937 (human DUSP6) and NP_446335 (rat DUSP6) sequences was 98.425% on an overlap of 381 amino acids.

## Example 5. Dusp6 gene expression in postmortem human tissue from controls and multiple sclerosis subjects.

### 5.1. Materials and methods.

Quantitative real-time RT-PCR studies were performed to evaluate if dusp6 expression was altered in the central nervous system of multiple sclerosis patients, as compared to control individuals matched by age and gender, who had died with no neurological disease symptoms.
Tissue samples originated from optic nerves were dissected out within 8 hours postmortem and snap frozen in liquid nitrogen. Total RNA was extracted with Trizol (Invitrogen, Paisley, UK) and cDNA was synthesized by reverse transcription (Applied Biosystems, Madrid, Spain) using random hexamers. Real-time quantitative RT-PCR was carried out in an ABI PRISM 7000 Sequence Detection System instrument (Applied Biosystems) using the primers SEQ ID NO:24 and SEQ ID NO:25 designed using the PrimerExpress software (Applied Biosystems). The amount of cDNA was calculated from the appropriate standard curve of a stock cDNA obtained from human cerebral cortex. Cyclophilin C, GAPDH, r18S, ubiquitin C, HRPT1 and 2-β-microglobulin housekeeping genes were used as endogenous references to normalize the variability in the initial quantities of total RNA, so that accurate comparison of gene expression levels could be made among the different samples. Target genes were normalized by means of a normalization factor, based on the geometric mean of multiple internal control genes (Vandesompele et al., 2002 Genome Biol 3:1-12.) obtained from the five internal control genes. The results are shown in figure 6; data are expressed as fold change in gene expression compared to the matched-controls. All results are expressed as mean ± SEM and statistical comparisons made by non paired, one tail Student's *t* test.

### 5.2. Results.

Quantitative RT-PCR analysis revealed that dusp 6 gene in all the samples analysed is expressed in variable amounts as compared to the five housekeeping genes used. In average, Multiple sclerosis patients samples had an increased expression of 43.83 ± 0.28 % over controls ($p=0.074$, $n=13$; Fig. 6).

### 5.3. Discussion.

The above described assays showed that the expression of dusp6 is increased in samples of optic nerve from multiple sclerosis patients. Because oligodendrocytes are the major cellular component of the optic nerve, the increased expression of dusp6 in the nerve most probably corresponds to higher transcript levels of this gene in these cells. This suggests that oligodendrocytes in the multiple sclerosis patients samples are at a higher risk of undergoing cell death, since dusp6 gene product is associated to cellular damage (Rossig et al., 2000, J. Biol. Chem. 275:25502-25507).

## Claims

1. An *in vitro* method for detecting the presence of demyelinating diseases in an individual, for determining the stage or severity of said diseases in the individual, or for monitoring the effect of the therapy administered to an individual presenting said diseases, comprising:

   a) detection and/or quantification of the DUSP6 protein, of the dusp6 gene mRNA, or of the corresponding cDNA in a sample of said individual, and
   b) comparison of the DUSP6 protein amount, of the dusp6 gene mRNA amount, or of the corresponding cDNA amount detected in a sample of an individual, with the DUSP6 protein amount, with the dusp6 gene mRNA amount, or with the corresponding cDNA amount detected in samples from control individuals or with normal reference values.

2. A method according to claim 1, wherein demyelinating diseases are, among others, multiple sclerosis, Devic's syndrome, Baló disease, Marchiafava-Bignami disease, central pontine myelinolysis, acute disseminated encephalomyelitis, or acute necrotizing hemorrhagic encephalomyelitis.

3. A method according to the previous claims, wherein said sample is of serum, urine, saliva, feces, or cerebrospinal fluid.

4. A method according to claim 3, wherein said serum, urine, saliva, feces, or cerebrospinal fluid sample to analyze is obtained by any conventional method, preferably surgical resection.

5. A method according to claim 1, wherein said sample to analyze is obtained from an individual who has not previously been diagnosed with a demyelinating disease.

6. A method according to claim 5, wherein said sample to analyze is obtained from an individual who has previously been diagnosed with a demyelinating disease, preferably multiple sclerosis.

7. A method according to claim 1, wherein said sample to analyze is obtained from an individual undergoing treatment, or who has been previously treated against a demyelinating disease, preferably multiple sclerosis.

8. A method according to claim 1, **characterized in that** it comprises carrying out an extraction of the sample, either for obtaining a protein extract or for obtaining an extract consisting of total RNA.

9. A method according to claim 8, **characterized in that** detection of the DUSP6 protein comprises a first step of contacting the protein extract of the sample with a composition of one or more specific antibodies against one or more epitopes of the DUSP6 protein, and a second step of quantifying the complexes formed by the antibodies and DUSP6 protein.

**10.** A method according to claim 9, **characterized in that** said antibodies comprise monoclonal, polyclonal antibodies, intact or recombinant fragments of them, "combibodies" and Fab or scFv antibody fragments, specific against the DUSP6 protein; these antibodies being human, humanized or of non-human origin.

**11.** A method according to claims 9 or 10, **characterized in that** for quantifying the complexes formed by the antibodies and the DUSP6 protein, techniques are used selected from the group formed by Western-blot, ELISA (Enzyme-Linked Immunosorbent Assay), RIA (Radioimmunoassay), Competitive EIA (Competitive Enzyme Immunoassay), DAS-ELISA (Double Antibody Sandwich-ELISA), immunocytochemical and immunohistochemical techniques, techniques based on the use of protein biochips or microarrays including specific antibodies, assays based on precipitation with colloidal gold in formats such as *dipsticks;* or by means of affinity chromatography techniques, ligand binding assays or lectin binding assays.

**12.** A method according to claim 8, **characterized in that** detection of mRNA or corresponding cDNA dusp6 gene comprises a first amplification step of the mRNA included in the total RNA extract, or of the corresponding cDNA synthesized by reverse transcription of the mRNA, and a second quantification step of the amplification product of the mRNA or cDNA of the dusp6 gene.

**13.** A method according to claim 12, **characterized in that** amplification is carried out in a qualitative or quantitative manner by means of RT-PCR using oligonucleotide primers, the sequences of the primers used to amplify the dusp6 gene sequence being SEQ ID NO:1 and SEQ ID NO:2, or any other primer pair amplifying dusp6 specifically.

**14.** A method according to claim 8, **characterized in that** detection is carried out with mRNA or corresponding cDNA specific probes of the dusp6 gene by means of techniques such as, for example, Northern-blot or Northern transfer.

**15.** A method according to claim 8, **characterized in that** mRNA detection is carried out by means of real time quantitative RT- PCR (Q-PCR).

**16.** The use of nucleotide or peptide sequences derived from the dusp6 gene for detecting *in vitro* the presence of a demyelinating disease in an individual, especially multiple sclerosis, for determining *in vitro* the stage or severity of said demyelinating diseases in the individual, or for monitoring in vitro the effect of the therapy administered to an individual presenting said demyelinating diseases.

**17.** An *in vitro* method for identifying and evaluating the efficacy of compounds for therapy of demyelinating diseases, preferably multiple sclerosis, comprising:

a) treating a primary culture of rat optic nerve oligodendrocytes with stimuli relevant to demyelinating diseases, preferably with excitotoxic stimuli such as Ampa or Kainate
b) detecting and quantifying changes in the dusp6 gene or DUSP6 protein expression in culture cells in response to said stimuli,
c) contacting the pure culture of stimulated oligodendrocytes obtained in step a) with the candidate compound under the conditions and for the time suitable for permitting them to interact,
d) detecting and quantifying the dusp6 gene or DUSP6 protein expression levels, and
e) comparing the expression levels obtained in step d) with the corresponding levels in pure cultures of stimulated oligodendrocytes not treated with the candidate compound.

**18.** The use of nucleotide or peptide sequences derived from the dusp6 gene in methods of search, identification, development and evaluation of efficacy of compounds for therapy of demyelinating diseases, preferably for multiple sclerosis.

**19.** An agent **characterized in that** it inhibits DUSP6 protein expression and/or activity, or **in that** it inhibits the lethal effects of induction of DUSP6 protein expression.

**20.** An agent according to claim 19 selected from the group formed by:

a) an antibody, or combination of antibodies, specific against one or more epitopes present in the DUSP6 protein, preferably a human or humanized monoclonal antibody; also being possible a fragment of the antibody, a single-chain antibody or an anti-idiotype antibody,
b) cytotoxic agents, such as toxins, molecules with radioactive atoms, or chemotherapeutic agents, which include, without limitation, small organic and inorganic molecules, peptides, phosphopeptides, antisense molecules, ribozymes, triple helix molecules, small interference RNA, double-strand RNA, etc., inhibiting DUSP6 protein expression and/or activity, such as, for example, the dusp6 specific antisense oligonucleotides SEQ ID NO:3 or SEQ ID NO:4, or any antisense oligonucleotide with an homology with said molecule exceeding 50%, or any dusp6 specific antisense oligonucleotide inhibiting its expression, and
c) DUSP6 protein antagonist compounds inhibiting one or more of the DUSP6 protein functions.

**21.** An agent according to claims 19 or 20 for the treatment of demyelinating diseases, especially multiple sclerosis.

**22.** Use of the agents according to claims 19 or 20 in the manufacturing of a drug for the treatment of demyelinating diseases, especially multiple sclerosis.

**23.** A pharmaceutical composition comprising a therapeutically effective amount of one or several agents according to claims 19 or 20, together with at least one pharmaceutically acceptable excipient.

**24.** A pharmaceutical composition according to claim 23, **characterized in that** it contains another active ingredient, preferably one which inhibits DUSP6 protein function.

FIGURE 1a

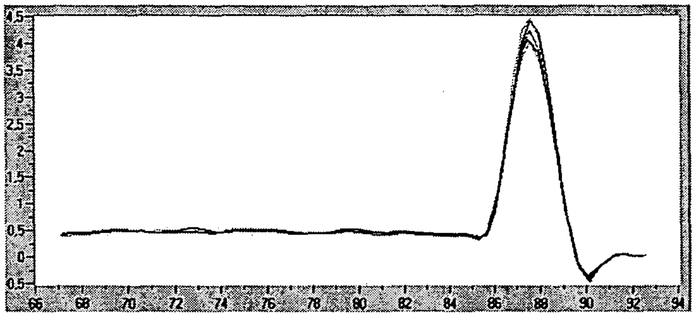


FIGURE 1b

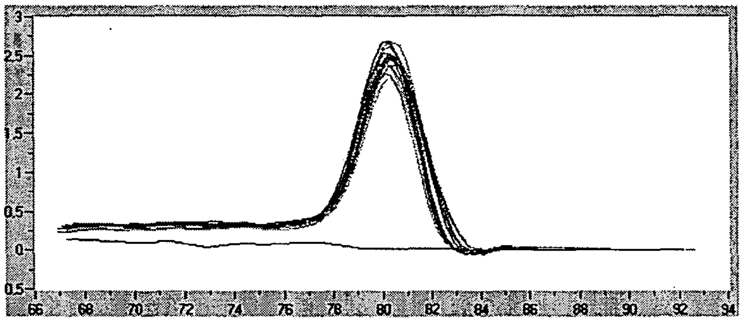

FIGURE 2

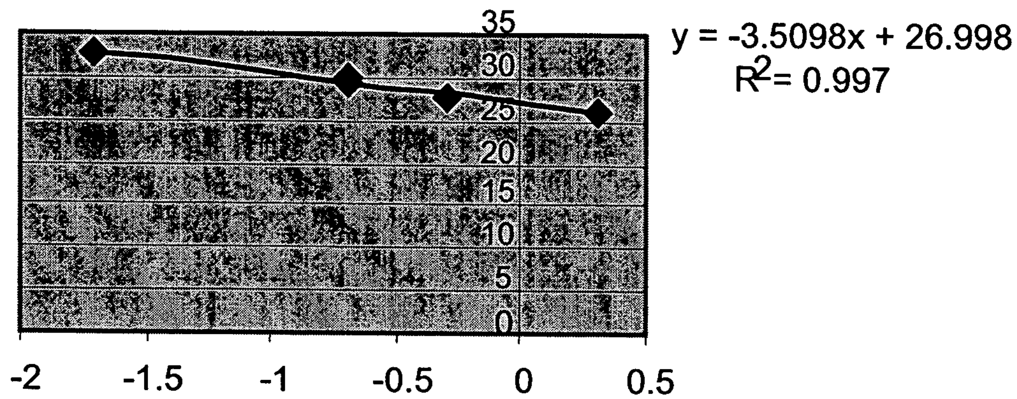

FIGURE 3

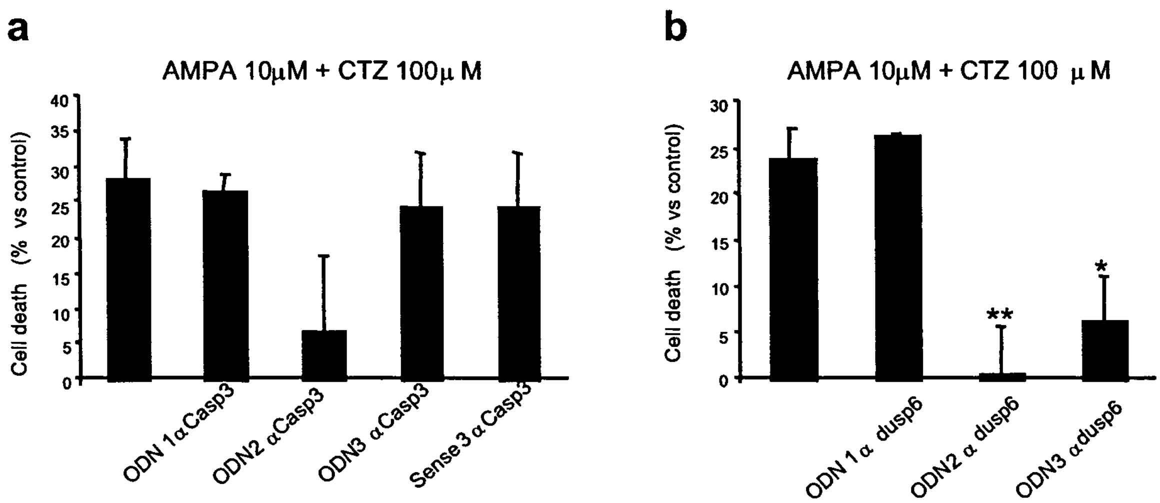
a
AMPA 10μM + CTZ 100μ M
Cell death (% vs control)
0
5
10
15
20
25
30
35
40
ODN 1αCasp3
ODN2 αCasp3
ODN3 αCasp3
Sense3αCasp3
b
AMPA 10μM + CTZ 100 μ M
Cell death (% vs control)
0
5
10
15
20
25
30
**
*
ODN 1α dusp6
ODN2 α dusp6
ODN3 αdusp6

**FIGURE 4**

```
86.713% identity (88.783% ungapped) in 1716 nt overlap
(172-1875:189-1876)
               150       160       170       180       190       200
human   CACTAAGAGCCAGCGCTGCAGCTGGTGCAGAGAGAACCTCCGGCTTTGACTTCTGTCTCG
                                      ::::   :  : :  ::: ::::::  :::
rat     GATTCATTGACTCTGAGAGTGATCTGGTGCAGAGGGACCACCGGCTTGGCTTCTG--TCG
       160       170       180       190       200       210

               210       220       230       240       250       260
human   TCTGCCCCAAGGCCGCTAGCCTCGGCTTGGGAAGGCGAGGCGGAATTAAACCCCGCTCCG
         :: ::: ::  :::::::: ::::::::::::    :::: :::: :::::: ::::::
rat     CCTTCCCTAA--CCGCTAGCTTCGGCTTGGGAA----AGGCCGAATCAAACCCGGCTCCG
         220         230       240           250       260       270

               270       280       290       300       310       320
human   AGAG-CGCACGTTCGCGCGCGGTGCGTCGGCCATTGCCTGCCCCGAGGGGCGTCTGGTAG
        :::: :: :  ::: : ::   ::: : ::::  :::::::: ::::::::::::: :::
rat     AGAGCCGGAGCTTCTCACGGCTTGC-TTGGCCTATGCCTGCCTCGAGGGGCGTCTGCTAG
               280       290        300       310       320

               330       340       350       360       370       380
human   GCACCCCGCCCTCTCCCGCAGCTCGACCCCCATGATAGATACGCTCAGACCCGTGCCCTT
        :::::::::: ::::: :::::::::::::::::::::::::::::::::::::::::::
rat     GCACCCCGCCTTCTCCTGCAGCTCGACCCCCATGATAGATACGCTCAGACCCGTGCCCTT
      330       340       350       360       370       380

               390       400       410       420       430       440
human   CGCGTCGGAAATGGCGATCAGCAAGACGGTGGCGTGGCTCAACGAGCAGCTGGAGCTGGG
        ::::::::::::::::::::::::::::::::::::::::::::::::::::::::::::
rat     CGCGTCGGAAATGGCGATCAGCAAGACGGTGGCGTGGCTCAACGAGCAGCTGGAGCTGGG
      390       400       410       420       430       440

               450       460       470       480       490       500
human   CAACGAGCGGCTGCTGCTGATGGACTGCCGGCCGCAGGAGCTATACGAGTCGTCGCACAT
        :::::: : ::::::::::::::::::::: ::::::::::: :::::::::::::::::
rat     CAACGAACAGCTGCTGCTGATGGACTGCCGACCGCAGGAGCTGTACGAGTCGTCGCACAT
      450       460       470       480       490       500

               510       520       530       540       550       560
human   CGAGTCGGCCATCAACGTGGCCATCCCGGGCATCATGCTGCGGCGCCTGCAGAAGGGTAA
        ::: :: :::::::::::::::::::::::::::::::::::::: ::::::::::: ::
rat     CGAATCTGCCATCAACGTGGCCATCCCGGGCATCATGCTGCGGCGTCTGCAGAAGGGCAA
      510       520       530       540       550       560

               570       580       590       600       610       620
human   CCTGCCGGTGCGCGCGCTCTTCACGCGCGGCGAGGACCGGGACCGCTTCACCCGGCGCTG
        :::::::::::::::::: ::::::::: ::::::::::::::::::: ::: :::::::
rat     CCTGCCGGTGCGCGCGCTATTCACGCGCTGCGAGGACCGGGACCGCTTTACCAGGCGCTG
      570       580       590       600       610       620

               630       640       650       660       670       680
human   TGGCACCGACACAGTGGTGCTCTACGACGAGAGCAGCAGCGACTGGAACGAGAATACGGG
         ::::::::::: ::::::::::::::::::: ::::::::::::::: ::::: :: ::
rat     CGGCACCGACACCGTGGTGCTCTACGACGAGAACAGCAGCGACTGGAATGAGAACACAGG
```

```
    630       640       650       660       670       680

                 690       700       710       720       730       740
human   CGGCGAGTCGTTGCTCGGGCTGCTGCTCAAGAAGCTCAAGGACGAGGGCTGCCGGGCGTT
        :: :::::: : ::::::::::::::::::: ::::: ::::::::::::::::::::
rat     TGGAGAGTCGGTCCTCGGGCTGCTGCTCAAGAAACTCAAAGACGAGGGCTGCCGGGCGTT
    690       700       710       720       730       740

                 750       760       770       780       790       800
human   CTACCTGGAAGGTGGCTTCAGTAAGTTCCAAGCCGAGTTCTCCCTGCATTGCGAGACCAA
        :::::: ::::::::::::::::::::::: ::::::::: ::::::: :::::::::::
rat     CTACCTTGAAGGTGGCTTCAGTAAGTTCCAGGCCGAGTTCGCCCTGCACTGCGAGACCAA
    750       760       770       780       790       800

                 810       820       830       840       850       860
human   TCTAGACGGCTCGTGTAGCAGCAGCTCGCCGCCGTTGCCAGTGCTGGGGCTCGGGGGCCT
        :::::::::::::: :::::::::: ::::: :::::::::::::: ::::::::::
rat     TCTAGACGGCTCGTGCAGCAGCAGCTCCCCGCCCTTGCCAGTGCTGGGACTCGGGGGCCT
    810       820       830       840       850       860

                 870       880       890       900       910       920
human   GCGGATCAGCTCTGACTCTTCCTCGGACATCGAGTCTGACCTTGACCGAGACCCCAATAG
        : ::::::::: ::::::::::::::::: :::::::::::::::::::::::::::
rat     GAGGATCAGCTCCGACTCTTCCTCGGACATTGAGTCTGACCTTGACCGAGACCCCAATAG
    870       880       890       900       910       920

                 930       940       950       960       970       980
human   TGCAACAGACTCGGATGGTAGTCCGCTGTCCAACAGCCAGCCTTCCTTCCCAGTGGAGAT
        :::::: ::::: ::::: :: :::::::::::::::::::::::::::: ::::::::
rat     TGCAACGGACTCCGATGGCAGCCCGCTGTCCAACAGCCAGCCTTCCTTCCCGGTGGAGAT
    930       940       950       960       970       980

                 990      1000      1010      1020      1030      1040
human   CTTGCCCTTCCTCTACTTGGGCTGTGCCAAAGACTCCACCAACTTGGACGTGTTGGAGGA
        ::::::::::: ::: ::::::::::::: ::::: :: :::::::::::::::: ::
rat     TTTGCCCTTCCTTTACCTGGGCTGTGCCAAGGACTCTACTAACTTGGACGTGTTGGAAGA
    990      1000      1010      1020      1030      1040

                1050      1060      1070      1080      1090      1100
human   ATTCGGCATCAAGTACATCTTGAACGTCACCCCCAATTTGCCGAATCTCTTTGAGAACGC
        :: ::::::::::::::::::::::::::::::::: :::: ::::: :::::::: ::
rat     GTTTGGCATCAAGTACATCTTGAACGTCACCCCCAATCTGCCCAATCTGTTTGAGAATGC
   1050      1060      1070      1080      1090      1100

                1110      1120      1130      1140      1150      1160
human   AGGAGAGTTTAAATACAAGCAAATCCCCATCTCGGATCACTGGAGCCAAAACCTGTCCCA
        ::: ::::: :: ::::::::::: :: ::::: :::::::::::::::::::::::::
rat     AGGGGAGTTCAAGTACAAGCAAATTCCTATCTCTGATCACTGGAGCCAAAACCTGTCCCA
   1110      1120      1130      1140      1150      1160

                1170      1180      1190      1200      1210      1220
human   GTTTTTCCCTGAGGCCATTTCTTTCATAGATGAAGCCCGGGGCAAGAACTGTGGTGTCTT
        :::::::::::::::::::::::::::::::::::::::: ::::: ::::::::::: :
rat     GTTTTTCCCTGAGGCCATTTCTTTCATAGATGAAGCCCGAGGCAAAAACTGTGGTGTCCT
   1170      1180      1190      1200      1210      1220
```

```
             1230      1240      1250      1260      1270      1280
human  GGTACATTGCTTGGCTGGCATTAGCCGCTCAGTCACTGTGACTGTGGCTTACCTTATGCA
       ::: ::::::::::: ::::: :::::::: ::::: ::::: :::::::::::::::::
rat    GGTGCATTGCTTGGCGGGCATCAGCCGCTCCGTCACGGTGACAGTGGCTTACCTTATGCA
    1230      1240      1250      1260      1270      1280

             1290      1300      1310      1320      1330      1340
human  GAAGCTCAATCTGTCGATGAACGATGCCTATGACATTGTCAAAATGAAAAAATCCAACAT
       :::::::::  :::: ::::::::::: :::::::::::::::::::: :: ::::::::
rat    GAAGCTCAACTTGTCCATGAACGATGCTTATGACATTGTCAAAATGAAGAAGTCCAACAT
    1290      1300      1310      1320      1330      1340

             1350      1360      1370      1380      1390      1400
human  ATCCCCTAACTTCAACTTCATGGGTCAGCTGCTGGACTTCGAGAGGACGCTGGGACTCAG
        :: :: ::::::::::::::::: :::::::: ::::: :: ::::: :::::::::::
rat    CTCTCCCAACTTCAACTTCATGGGCCAGCTGCTTGACTTTGAAAGGACCCTGGGACTCAG
    1350      1360      1370      1380      1390      1400

             1410      1420      1430      1440      1450      1460
human  CAGCCCATGTGACAACAGGGTTCCAGCACAGCAGCTGTATTTTACCACCCCTTCCAACCA
       :::::: ::::::::  : :: :: ::::::::::: :: :: ::: : :: ::::::::
rat    CAGCCCCTGTGACAATCGTGTCCCCGCACAGCAGCTCTACTTCACCGCGCCCTCCAACCA
    1410      1420      1430      1440      1450      1460

             1470      1480      1490      1500      1510      1520
human  GAATGTATACCAGGTGGACTCTCTGCAATCTACGTGAAAGACCCCACACCCCTCCTTGCT
       :::::: ::::: :::::::: :::::::::::::::::: : :: ::::  :::: ::
rat    GAATGTCTACCAAGTGGACTCCCTGCAATCTACGTGAAAGGCACC-CACCTTTCCTAGCC
    1470      1480      1490      1500      1510       1520

             1530      1540      1550       1560      1570
human  GGAATGTGTCTGGCCCTTCAGCAGTTTCTCTT-GGCAGCATCAGCTGGGCTGCTTTCTTT
       :: : ::::::  :  : :  ::::::::::: :::::::::  :  :::::::::::::
rat    GGGA-GTGTCT--CATTCCTTCAGTTTCTCTTGGGCAGCATCGACCAGGCTGCTTTCTTT
     1530         1540      1550      1560      1570      1580

    1580      1590       1600      1610      1620      1630
human  GTGTGTGGCCCCAGGTGTC-AAAATGACACCAGCTGTCTGTACTAGACAAGGTTACCAAG
       ::::::::::::::::::: :::::: ::::::::::::::: ::::::::::: :::::
rat    GTGTGTGGCCCCAGGTGTCAAAAATGTCACCAGCTGTCTGTATTAGACAAGGTTGCCAAG
        1590      1600      1610      1620      1630      1640

     1640      1650      1660      1670           1680      1690
human  TGCGGAATTGGTTAATACTAACAGAGAGATTTGCTCCATTC-----TCTTTGGAATAACA
       :::  ::::::::: :::  :  ::::::::::::::::::     : :::::::  :::
rat    TGCAAAATTGGTTATTACGGAGGGAGAGATTTGCTCCATTCATTGTTTTTTGGAAGGACA
        1650      1660      1670      1680      1690      1700

          1700      1710      1720       1730      1740      1750
human  GGACATGCTGTATAGATACAGGCAGTAGGTTTGC-TCTGTACCCATGTGTACAGCCTACC
       ::  ::::::: ::::: :::::: ::::::::: : :::::::       :::::::::
rat    GGGTATGCTGTCTAGATCCAGGCAATAGGTTTGCTTTTGTACCC-------CAGCCTACC
        1710      1720      1730      1740             1750

           1760      1770      1780         1790      1800
human  CATGCAGGGACTGGGATTCGAGGACTTCCAG---GCGCATAGGGTAGAACCAAATGATAG
```

```
         :: :::::::::::   ::      : :::::   : :  ::::  : ::  :   :   :
rat      CAAGCAGGGACTGGACCTC-----CATCCAGATAGAGGGTAGGACA-AAGGAGCCG--GG
       1760      1770            1780      1790        1800        1810

    1810      1820      1830      1840      1850      1860
human    GGTAGGAGCATGTGTTCTTTAGGGCCTTGTAAGGCTGTTTCCTTTTGCATCTGGAACTGA
         : ::::::::::::::: ::::::::   :: ::::::::::: :::::::::::::  :
rat      GATAGGAGCATGTGTTCCTTAGGGCCACATATGGCTGTTTCCTGTTGCATCTGGAACCAA
               1820      1830      1840      1850      1860      1870

    1870      1880      1890      1900      1910      1920
human    CTATATAATTGTCTTCAAGTGAAGACTAATTCAATTTTGCATATAGAGGAGCCAAAGAGA
         ::::::
rat      CTATATTGTCTTCAGTGAAGACTGATTCAACTTTGCGTATAGTGGAGCCAAAGAGATTTT
               1880      1890      1900      1910      1920      1930
```

**FIGURE 5**

```
98.425% identity (98.425% ungapped) in 381 aa overlap (1-381:1-381)

               10        20        30        40        50        60
human  MIDTLRPVPFASEMAISKTVAWLNEQLELGNERLLLMDCRPQELYESSHIESAINVAIPG
       ::::::::::::::::::::::::::::::::.:::::::::::::::::::::::::::
rat    MIDTLRPVPFASEMAISKTVAWLNEQLELGNEQLLLMDCRPQELYESSHIESAINVAIPG
               10        20        30        40        50        60

               70        80        90       100       110       120
human  IMLRRLQKGNLPVRALFTRGEDRDRFTRRCGTDTVVLYDESSSDWNENTGGESLLGLLLK
       ::::::::::::::::::: ::::::::::::::::::::.::::::::::::.::::::
rat    IMLRRLQKGNLPVRALFTRCEDRDRFTRRCGTDTVVLYDENSSDWNENTGGESVLGLLLK
               70        80        90       100       110       120

              130       140       150       160       170       180
human  KLKDEGCRAFYLEGGFSKFQAEFSLHCETNLDGSCSSSSPPLPVLGLGGLRISSDSSSDI
       :::::::::::::::::::::::.::::::::::::::::::::::::::::::::::::
rat    KLKDEGCRAFYLEGGFSKFQAEFALHCETNLDGSCSSSSPPLPVLGLGGLRISSDSSSDI
              130       140       150       160       170       180

              190       200       210       220       230       240
human  ESDLDRDPNSATDSDGSPLSNSQPSFPVEILPFLYLGCAKDSTNLDVLEEFGIKYILNVT
       ::::::::::::::::::::::::::::::::::::::::::::::::::::::::::::
rat    ESDLDRDPNSATDSDGSPLSNSQPSFPVEILPFLYLGCAKDSTNLDVLEEFGIKYILNVT
              190       200       210       220       230       240

              250       260       270       280       290       300
human  PNLPNLFENAGEFKYKQIPISDHWSQNLSQFFPEAISFIDEARGKNCGVLVHCLAGISRS
       ::::::::::::::::::::::::::::::::::::::::::::::::::::::::::::
rat    PNLPNLFENAGEFKYKQIPISDHWSQNLSQFFPEAISFIDEARGKNCGVLVHCLAGISRS
              250       260       270       280       290       300

              310       320       330       340       350       360
human  VTVTVAYLMQKLNLSMNDAYDIVKMKKSNISPNFNFMGQLLDFERTLGLSSPCDNRVPAQ
       ::::::::::::::::::::::::::::::::::::::::::::::::::::::::::::
rat    VTVTVAYLMQKLNLSMNDAYDIVKMKKSNISPNFNFMGQLLDFERTLGLSSPCDNRVPAQ
              310       320       330       340       350       360

              370       380
human  QLYFTTPSNQNVYQVDSLQST
       :::::.:::::::::::::::
rat    QLYFTAPSNQNVYQVDSLQST
              370       380
```

FIGURE 6

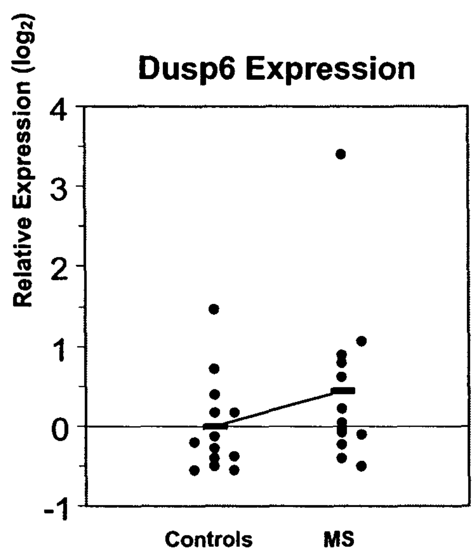
Dusp6 Expression
Relative Expression (log2)
4
3
2
1
0
-1
Controls
MS

European Patent Office

# PARTIAL EUROPEAN SEARCH REPORT

which under Rule 45 of the European Patent Convention shall be considered, for the purposes of subsequent proceedings, as the European search report

Application Number
EP 03 38 0310

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 03/099781 A (BOEHRINGER INGELHEIM PHARMA) 4 December 2003 (2003-12-04) p. 9, lines 1 - 9, p. 15, lines 9 - 20, claims 17 - 20,, Fig. 1 --- | 18-24 | G01N33/573<br>C12N9/16 |
| X | WO 03/003977 A (CHEN HONG ;XU HAIYAN (US); MILLENNIUM PHARM INC (US)) 16 January 2003 (2003-01-16) p. 4, line 5 - p. 6, line 15, p. 13, line 32 - p. 16, line 17, p. 28, line 21 - p. 31, line 30, p. 36, line 21 - p. p. 40, line 16, p. 68, line 24 - p. 78, line 27, claims 1 - 7, Seq Id Nos. 1-3 --- | 19-24 | |
| | -/-- | | |

TECHNICAL FIELDS SEARCHED (Int.Cl.7)

G01N
C12N

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

3

| Place of search | Date of completion of the search | Examiner |
|---|---|---|
| MUNICH | 22 June 2004 | Hoesel, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

# PARTIAL EUROPEAN SEARCH REPORT

Application Number
EP 03 38 0310

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | TAKAKI MANABU ET AL: "Two kinds of mitogen-activated protein kinase phosphatases, MKP-1 and MKP-3, are differentially activated by acute and chronic methamphetamine treatment in the rat brain" JOURNAL OF NEUROCHEMISTRY, vol. 79, no. 3, November 2001 (2001-11), pages 679-688, XP002285424 ISSN: 0022-3042 * abstract * --- | 1-24 | |
| A | FURUKAWA TORU ET AL: "Potential tumor suppressive pathway involving DUSP6/MKP-3 in pancreatic cancer." AMERICAN JOURNAL OF PATHOLOGY, vol. 162, no. 6, June 2003 (2003-06), pages 1807-1815, XP002285425 ISSN: 0002-9440 (ISSN print) abstract, p. 1814, col. 1, lines 23 - end of column --- | 1-24 | TECHNICAL FIELDS SEARCHED (Int.Cl.7) |
| A | SORIANO M A ET AL: "PARALLEL GENE EXPRESSION MONITORING USING OLIGONUCLEOTIDE PROBE ARRAYS OF MULTIPLE TRANSCRIPTS WITH AN ANIMAL MODEL OF FOCAL ISCHEMIA" JOURNAL OF CEREBRAL BLOOD FLOW AND METABOLISM, RAVEN PRESS, LTD., NEW YORK, NY, US, vol. 20, no. 7, July 2000 (2000-07), pages 1045-1055, XP009012666 ISSN: 0271-678X * abstract * --- -/-- | 1-24 | |

3

EPO FORM 1503 03.82 (P04C10)

European Patent Office

# PARTIAL EUROPEAN SEARCH REPORT

Application Number
EP 03 38 0310

| Category | Citation of document with indication, where appropriate, of relevant passages | Relevant to claim | CLASSIFICATION OF THE APPLICATION (Int.Cl.7) |
|---|---|---|---|
| A | MUDA M ET AL: "MKP-3, A NOVEL CYTOSOLIC PROTEIN-TYROSINE PHOSPHATASE THAT EXEMPLIFIES A NEW CLASS OF MITOGEN-ACTIVATED PROTEIN KINASE PHOSPHATASE" JOURNAL OF BIOLOGICAL CHEMISTRY, AMERICAN SOCIETY OF BIOLOGICAL CHEMISTS, BALTIMORE, MD, US, vol. 271, no. 8, 23 February 1996 (1996-02-23), pages 4319-4326, XP000611987 ISSN: 0021-9258 * abstract; figure 1 * --- | 1-24 | |
| T | SOTO A ET AL: "Excitotoxic insults to the optic nerve alter visual evoked potentials." NEUROSCIENCE, vol. 123, no. 2, 23 January 2004 (2004-01-23), pages 441-449, XP001182129 ISSN: 0306-4522 * the whole document * ----- | 1-24 | TECHNICAL FIELDS SEARCHED (Int.Cl.7) |

DOCUMENTS CONSIDERED TO BE RELEVANT

3

EPO FORM 1503 03.82 (P04C10)

European Patent Office

**INCOMPLETE SEARCH SHEET C**

Application Number
EP 03 38 0310

Claim(s) searched incompletely:
19-24

Reason for the limitation of the search:

Present claims 19 - 24 relate to compounds defined by reference to a desirable characteristic or property, i.e. inhibition of MKP-3/DUSP6 expression or activity.

The claims cover all agenTs having this characteristic or property, whereas the application provides support within the meaning of Article 84 EPC and/or disclosure within the meaning of Article 83 EPC for only limited classes of such compounds. In the present case, the claims so lack support, and the application so lacks disclosure, that a meaningful search over the whole of the claimed scope is impossible. Independent of the above reasoning, the claims also lack clarity (Article 84 EPC). An attempt is made to define a compound in terms of a result to be achieved. Again, this lack of clarity in the present case is such as to render a meaningful search over the whole of the claimed scope impossible. Consequently, the search has been carried out for those parts of the claims which appear to be clear, supported and disclosed, namely those parts relating to antisense nucleic acids or anti MKP-3/DUSP6 antibodies.

## ANNEX TO THE EUROPEAN SEARCH REPORT ON EUROPEAN PATENT APPLICATION NO. EP 03 38 0310

This annex lists the patent family members relating to the patent documents cited in the above-mentioned European search report.
The members are as contained in the European Patent Office EDP file on
The European Patent Office is in no way liable for these particulars which are merely given for the purpose of information.

22-06-2004

| Patent document cited in search report | Publication date | Patent family member(s) | Publication date |
|---|---|---|---|
| WO 03099781 A | 04-12-2003 | WO 03099781 A2<br>US 2004014111 A1 | 04-12-2003<br>22-01-2004 |
| WO 03003977 A | 16-01-2003 | WO 03003977 A2<br>US 2003022857 A1 | 16-01-2003<br>30-01-2003 |

EPO FORM P0459

For more details about this annex : see Official Journal of the European Patent Office, No. 12/82